(19) Europäisches Patentamt / European Patent Office / Office européen des brevets

(11) **EP 1 716 837 B1**

(12) **EUROPEAN PATENT SPECIFICATION**

(45) Date of publication and mention
of the grant of the patent:
**07.06.2017 Bulletin 2017/23**

(51) Int Cl.:
*A61K 8/42* *(2006.01)*  *A61Q 19/00* *(2006.01)*
*A61K 8/02* *(2006.01)*

(21) Application number: **05004306.6**

(22) Date of filing: **28.02.2005**

(54) **Use of a volatile cooling sensate on fibrous tissues to provide a sensation of rhinological decongestion**

Verwendung von einem Kältegefühl erzeugenden flüchtigen Material auf faserigen Stoffen um eine rhinologische Dekongestion zu erreichen

Utilisation d'une composé volatil produisant une sensation de froideur sur un tissage fibreux pour une sensation de décongestion rhinologique

(84) Designated Contracting States:
**AT BE BG CH CY CZ DE DK EE ES FI FR GB GR HU IE IS IT LI LT LU MC NL PL PT RO SE SI SK TR**

(43) Date of publication of application:
**02.11.2006 Bulletin 2006/44**

(73) Proprietor: **THE PROCTER & GAMBLE COMPANY Cincinnati, OH 45202 (US)**

(72) Inventor: **Kleinwaechter, Joerg 65719 Hofheim (DE)**

(74) Representative: **Kremer, Véronique Marie Joséphine Procter & Gamble Service GmbH IP Department Frankfurter Strasse 145 61476 Kronberg im Taunus (DE)**

(56) References cited:
**EP-A- 1 250 941    EP-A- 1 332 772
EP-A- 1 408 155    WO-A-02/41861
WO-A-96/19204**

EP 1 716 837 B1

**Description**

Field of Invention

The present invention relates to tissues useful in providing fibrous tissue products for skin contact, such as facial tissue, bathroom tissue, toilet tissue, disposable handkerchiefs comprising a volatile cooling sensate effective on human skin by causing a cold feeling to provide sensation of rhinological decongestion without directly causing a change in skin temperature. Preferably the cooling sensate is provided as a component of a lotion composition, which in turn is preferably provided as discrete deposits of a localized high basis weight. The cooling effect is perceived as easier breathing thus providing the sensation of reduced rhinological congestion.

Background of the Invention

Fibrous tissues useful in providing tissue products for skin contact are most frequently simple paper tissues, which find extensive use in modem society and are well known in the art. They are sometimes called paper webs or sheets, tissues, tissue layers, paper plies or paper tissue webs, and products made there from, such as paper handkerchiefs, paper kitchen towels or bath tissues, toilet paper or facial tissues.

Paper tissues, or more generally fibrous tissues of the present context, are generally made by the layering of fibers, mostly cellulose fibers, in a wet form, onto a screen, with the addition of various additives or other ingredients, optionally including other, natural or synthetic fibers, followed by a drying step. Other process steps, before, during or after the above-mentioned paper tissue making steps are targeted at giving the desired properties to the tissue. Converting steps are aimed at creating a finished product from the tissue(s).

Products made from fibrous tissues can be made by the association of multiple layers of tissues, also called plies, or can comprise a single tissue layer (single ply products). Those plies can be combined and held together in multiple ways to form the finished product, for example by embossing of the multi-ply structure or/and by gluing. The finished products are herein referred to as paper tissue products or fibrous tissue products. Finished products made of more than one ply have internal tissue (or ply) surfaces, inwardly orientated, and 2 external surfaces, outwardly orientated.

It has long been recognized that important physical attributes of these paper tissue products are their strength and thickness/bulkiness, their softness and smoothness, and their absorbency. Softness and smoothness relate to the tactile sensation perceived by the consumer when holding a particular product, rubbing it across the skin, or crumpling it within the hands.

Relatively thick and yet soft disposable paper products, namely in the form of paper handkerchiefs, are known. For example, Tempo™, sold by The Procter & Gamble Company, is a multi-ply paper product experienced as thick and soft and having a caliper of about 0.3 mm. A high caliper conveys the idea of high dry and wet strength to the consumer. A high wet strength, also referred to as wet burst strength, in particular prevents tearing or bursting, which for a paper handkerchief in turn results in contamination of the user's hand with mucus or other body fluids.

A common way to enhance the smoothness of the tissue surface is to calender the material. Another way to improve the sensation of smoothness perceived by the users of paper tissue products, such as handkerchiefs, is to complement the composition of the tissue with some additives during the paper-making phase and/or during the converting phase. Those additives can have the effect of smoothening the tissue in a way that makes the user feel it more soft or smooth. Alternatively or additionally some additives have an effect on the skin of the user touching or using the paper tissue product, e.g. smoothening of the skin, hydration of the skin. These later effects are usually obtained through a partial transfer of the additives onto the skin during usage, thus prolonging the effect of the additives on the skin beyond the period of contact between the paper-tissue product and skin. Smoothening lotion is a term to describe usually those additives which (a) can have a softening effect on the tissue material, (b) preferably an effect on the skin, and (c) are partially transferred onto the skin of the user during use. Conventionally smoothening lotions are applied on a native tissue surface at the converting stage during manufacturing of paper tissue products (as opposed to the paper-making stage). Smoothening lotions are usually of hydrophobic nature or contain hydrophobic compounds. The presence of the lotion at the surface of the paper tissue product can have adverse effects on the properties: First, the masking of the hydrophilic tissue surface can reduce the absorbency or the speed of absorbency. Second, the lotion can migrate from the tissue surface into the structure making the tissue less hydrophilic and reducing the lotion available at the surface to deliver the smoothening benefits to the skin.Increasing the amount of lotion can in turn create an excess of lotion on the freshly produced tissues thus triggering a greasy feeling during use and further reducing the absorbency. It is hence desirable to provide a high amount of lotion available on the surface for transferability while concurrently maintaining a high absorbency of the tissue. Even if such problems of the smoothening of the skin of users of tissue products are addressed there remain improvement aspects for tissue products, especially during high frequency usage conditions e.g. when a user suffers from a cold or allergy induced runny nose and breathing difficulties often together with other discomfort referred to as rhinological discomfort. Relieving e.g. cold or allergy induced symptoms, whether by physiological reaction, by sensory effects on the skin of the user, perception of such reaction or perception of such effects, or combinations thereof, as a result of usage of conventional tissue products would be highly desirable. Thereby also the length of rhinological discomfort and the amount of tissue product required by the user can be reduced, with the added benefit of a direct reduction of the time for transmitting viral or bacterial causes of such discomfort. This follows the well known medical principle that fast relieve also reduces the contamination probability.

A cooling sensate, known for use in the disposable handkerchief context is menthol or camphor. However, despite their effective functionality it has been found to cause with many users an olfactory dislike, which has prevented widespread usage/acceptance of these sensates. Menthol and camphor are also suspected to have caused irritations especially on fair skin, at least at high dosages.

Accordingly, there is a need to provide a fibrous tissue exhibiting a physical and/or sensory rhinological discomfort relieve, preferably in combination with a relatively high amount of lotion available at the surface of the tissue in the form of discrete deposits protruding from the surface of the tissue.

Further, there is a need for providing such a solution in an efficient, safe, affordable way, especially by providing the discomfort relieve with high efficacy, e.g. by ensuring good transferability, long exposure to the discomfort relieve means without unacceptable loss of other important tissue attributes, such as absorbency, strength or softness of tissues.

### Summary of the Invention

In order to address the issues related to the state of the art and advance the non-medical cold relieve technology, the present invention provides a fibrous tissue for use in tissue products for skin contact, such as facial tissue, bathroom tissue, toilet tissue or disposable handkerchiefs. Besides fibers suitable for the final intended use the tissue comprises a volatile cooling sensate (this needs to be olfactory acceptable and hence excludes menthol and camphor at least at levels detectable by smell), which is effective on human mucous skin by causing a cold feeling to provide sensation of rhinological decongestion without directly causing a change in skin temperature.

It is understood that in the context of handkerchiefs the cold feeling is experienced as increased evaporation, which in turn is equated with an easier breathing. , It should be noted that the present invention is directed to tissues used for providing facial tissues, bathroom tissues, toilet tissues and disposable handkerchiefs. Albeit it may appear unconventional for bathroom tissues and toilet tissues, considering the benefits contemplated by the present invention, such tissue products are frequently used by consumers as facial tissues, bathroom tissues or disposable handkerchiefs. Hence use of fibrous tissues for such products is a logical extension of the more conventional use.

The volatile cooling sensate is 2-Isopropyl-N,2,3-trimethylbutyramide. Hence use of fibrous tissues for such products is a logical extension of the more conventional use. It is further preferred that a transferable lotion is present in addition to the volatile cooling sensate.

In another aspect of the invention the cooling sensate can be provided as a compound in a transferable lotion on the fibrous tissue. Besides the benefits of longevity of the effect of the cooling sensate, due to being transferred to the skin, is the reduced olfactory recognition of the smell of cooling sensates, thus allowing also the use of a higher amount of menthol as a compound of the cooling sensate provided it remains at levels substantially undetectable by smell.

To ensure best efficacy of the lotion as to its transferability, lotion smoothening effect and cooling sensate transferability, it is present in substantially discrete deposits on at least a surface of said tissue and said lotion basis weight in said deposits is at least 11 g/sqm" preferably at least 15 g/sqm, more preferably at least 25g/sqm while the basis weight of lotion calculated on the overall tissue surface is equal or less than 9 g/sqm, preferably less than 6g/sqm, more preferably less than 3g/sqm. In particular preferred embodiments there are at least 2 deposits per square cm of tissue.

In particular when used in a facial tissue, bathroom tissue or disposable handkerchief the fibrous tissue comprises at least 50%, preferably 80%, more preferably 90% of cellulose fibers by weight of the fibers of the fibrous tissue. In order to address the polarizing effect (some like it, some hate it) of the olfactory recognition of some of the potential cooling sensate materials, notably menthol, usage of amounts below the olfactory recognition have been found to be particular desirable in handkerchiefs or facial tissues. Such usage is also referred to as subliminal usage.

### Detailed Description of the Invention

The present invention provides a fibrous tissue adapted for use in tissue products such as facial tissues, bathroom tissues, toilet tissues and disposable handkerchiefs comprising a volatile cooling sensate. In particular the tissue should also exhibit a high level of surface smoothness and softness, high absorbency, a high strength and a high bulkiness. Preferably the volatile cooling sensate is provided as a compound of a lotion applied in discrete deposits to the external surface of the tissue or of the tissue product.

Fibrous Tissue and tissue product

The terms "fibrous tissue" and "tissue" are referred to interchangeably herein and prominently includes tissue paper. Besides the fibers used in conventional tissue paper materials, which are substantially provided from cellulose in the form of wood pulp fibers and some papermaking additives, the fibrous tissue according to the present invention may comprise as well some, a substantial quantity, or even only fibers of a different nature. The fibers utilized for the present invention will preferably include fibers derived from wood pulp. Other cellulose fibers, such as cotton linters, bagasse, etc., can be utilized and are intended to be within the scope of this invention. Non-cellulose fibers such as those including starch and other polysaccharides, synthetic fibers, such as rayon, polyethylene, and polypropylene fibers can also be utilized alone or in combination with natural cellulose fibers. One exemplary polyethylene fiber that can be utilized is Pulpex®, available from Hercules, Inc. (Wilmington, Del.). Applicable wood pulps include chemical pulps, such as Kraft, sulfite, and sulfate pulps, as well as mechanical pulps including, for example, groundwood, thermo-mechanical pulp and chemically modified thermo-mechanical pulp. In addition to such fibers, a tissue making furnish used to make tissue structures can have other components or materials added thereto as known in the art. The types of additives desirable will be dependent upon the particular end use of the tissue sheet contemplated. For example, in tissue products such as toilet tissue, bathroom tissue, facial tissues or disposable handkerchiefs and other similar products, high wet strength is a desirable attribute. Thus, it is often desirable to add to the tissue making furnish chemical substances known in the art as "wet strength" resins.

The present invention is particularly useful with tissue paper in general, including but not limited to conventionally felt-pressed tissue paper; high bulk pattern densified tissue paper; and high bulk, un-compacted tissue paper and through-air dried tissue paper. Included in the tissue paper definition are dry laid substrates but preferred are the conventional wet laid tissue papers. The tissue paper can be of a homogenous or multi-layered construction. Tissue products made fibrous tissues can be of a single-ply or multi-ply construction. Tissue products preferably have a basis weight of between about 10 $g/m^2$ and about 65 $g/m^2$, and density of about 0.6 $g/cm^3$ or less. More preferably, the basis weight will be about 40 $g/m^2$ or less for facial tissues, and 60+/-10 $g/m^2$ for handkerchiefs and the density will be about 0.3 g/cc or less. For measuring the density of tissue paper see Column 13, lines 61-67, of U.S. Pat. No. 5,059,282 (Ampulski et al), issued Oct. 22, 1991, which describes how the density of tissue paper is measured. Unless otherwise specified, all amounts and weights relative to the paper are on a dry basis.

The "tissue products" of this invention are the finished products such as facial tissues, bathroom tissues, toilet tissues and disposable made from one or multiple plies of the above described fibrous tissues. Each ply of a multiply product can be made of different material or can have been manufactured in different ways. As used herein, the term "single-ply tissue product" means that it is comprised of one ply of tissue; the ply can be substantially homogeneous in nature or it can be a multi-layered tissue paper web. As used herein, the term "multi-ply tissue product" means that it is comprised of more than one ply of tissue. The plies of a multi-ply tissue product can be substantially homogeneous in nature or they can be multi-layered tissues.

**Cooling Sensate**

According to the present invention the tissue comprises as an essential component a volatile cooling sensate able to convey a cold/fresh perception to the user of such tissue without actually creating a direct reduction of temperature on the skin. The volatile cooling sensate is 2-Isopropyl-N,2,3-trimethylbutyramide.

By 'perception' it is meant the result perceived by the nervous central system of a user, which is a multi-step process initiated by the stimulation of sensory thermo receptors on/in the skin especially inside the nose and/or mucosal surface of said wearer. More details on such receptors are available in literature. An example of reference on thermo-receptors is:" Thermal sensation and thermo-receptors in man" by Herbert Hensel, M D, published by Charles C. Thomas in 1982.

The tissue products of the present invention comprise an amount of the volatile cooling sensate sufficient to reach and stimulate the receptors in the areas of the skin and/or mucosal surfaces such that the desired perception is created. The amount of sensate in each product will vary as the degree and longevity of the sensation varies from sensate to sensate and based on the way the sensate is applied to the tissue incorporated in the product. For the preferred sensates of the present invention an amount of at least 0.005 % of the tissue weight, preferred at least 0.01 % of the tissue weight has been found acceptable. If the sensate is incorporated with other materials, especially as a compound of a lotion on the tissue then amounts of at least 0.05% by weight of the lotion, preferably 0.1 1 % by weight of the lotion have been found to work satisfactory. In some embodiments of the present invention the sensate can also be encapsulated, complexed or stabilized in other forms usual in the art.

The sensate for use herein is a volatile cooling sensate, which is able to stimulate thermo-receptors (i.e., hot or cold sensory receptors), without the need to create direct temperature change on the skin. The cooling sensate suitable for use herein include cooling sensates being able to penetrate the skin barrier and for which the cooling effect (herein also referred to as freshness effect) is a physiological effect due to the direct action of the sensate on the nerve endings responsible for the detection of cold without the occurrence of temperature change. Due to the persistence of the stimuli a long lasting freshness/cooling sensation is delivered.

It is to be understood herein that the freshness/cooling sensation is personnel to a given individual. It must be admitted that skin tests are somewhat subjective, some individuals experiencing a greater or lesser freshness/cooling sensation than others when subjected to the same test. This perception depends on the density of thermo-receptors on skin and on the skin thickness. Typically it is observed that the thinner the skin is the more intense is the cooling sensation. Without to be bound by any theory, it is believed that the thinner the skin is, the more rapid is the penetration of the cooling agent through the skin and higher is the absorption level thereof.

Studies performed on cooling agent activity have showed that four features of the molecular structure of the cooling agents are particularly important to deliver cooling sensation. Reference is made to H.R. Watson et al., Journal of the Society of Cosmetic Chemist,Vol.29, p185-200, 1978 and all cooling sensate information disclosed therein.

Suitable cooling sensates for use herein conventionally posses some of the following properties:

- a hydrogen binding capability;
- a compact hydrocarbon skeleton to allow receptors to 'recognize' the sensate;
- a balance between their hydrophilic and hydrophobic parts for both delivering cooling properties and able them to penetrate the biological membrane such as outer skin layers- The most common method of determining this balance is to use the Hansch log P value - the coefficient of water and n-octanol distribution according to Hansch. The log P value is acknowledged as being a crucial factor in a substance's pharmacological activity - especially as regards how it is transported through skin. The log p value of preferred cooling sensates for use herein generally lies between 2 and 3;
- a molecular weight of between 50 and 350 g/mol.

Without wishing to be bound by theory, it is speculated that suitable cooling sensates for use herein are those being able to penetrate through the skin surface and depolarize (clear the potential differential between the inside and outside nervous cell membranes by blocking calcium ion exchange) the membrane of cold receptors. The perception of freshness sensation is the result of the depolarization. More particularly, it is believed that due to binding calcium properties of the cooling agents, the equilibrium between the concentration of calcium ion outside and inside the nervous cell membrane is disturbed. In other words, by reducing the calcium ion level outside the nervous cell membrane, the membrane is depolarized, resulting thereby in increased discharge rate of nerve fibers and hence transfer of electrical stimuli to the central nervous system.

A well known cooling sensate is menthol. Menthol is preferably not used herein as the cooling agent, although menthol is known to provide cooling sensation, it suffers the disadvantage of having a strong mint odor, being a known irritant to skin at high concentration and strongly sublimating at room temperature. In particular the high sublimation rate causes the problem that tissues comprising menthol either have a strong mint smell or if the smell is acceptable the majority of the menthol has already vaporized prior to use of the tissue (or the menthol was never present in an objectionable quantity).

Hence menthol as a substance is not included in the useful cooling sensates according to the present invention when used as a neat substance. However if menthol is incorporated into a matrix of other materials, e.g. as a compound in a lotion composition where its smell is subliminal, then its use is considered optional as an additional cooling sensate together with another cooling sensate according to the present invention. More generally it is preferred according to the present invention that there is substantially no olfactory recognition of the cooling sensate materials when used on tissues incorporated in particular in handkerchiefs or facial tissues. Such usage is also referred to as subliminal usage and can be achieved by selection of the cooling sensate as such but also by usage of low amounts of the sensate.

Particularly well known cooling sensate agents include ketals, carboxamides, cyclohexyl derivatives and/or cyclohexanol derivatives. Such and other cooling sensates are described in detail e.g. in EP-A-1.250.940 and EP-A-1.250.941, US-A-5.451.404, US-A-5.266.592, DE-A-2.608.226, and DE-A-2.458.562.

Sensates according to the present invention are: 2-Isopropyl-N,2,3-trimethylbutyramide, commercially available from e.g. Millennium Chemicals of Lyondell in Houston, Texas USA, under the trade name WS-23, or from Qaroma, BAYTOWN, TEXAS USA, under the trade name ICE 1000. This material has a vapour pressure of about 0.53 Pascal (equivalent to 0.004mmHg at about 20°C).

'Volatility' according to the present invention is considered to be present for substances having a liquid or sublimation vapour pressure of the pure substance at 20 °C of at least about 0.013 Pascal (0.0001mmHg), preferably at least 0.1 Pascal. Vapour pressure can be measured according to ASTM Standard E1194-01 of Nov 2001. In preferred embodiments according to the present invention an upper limit for the vapour pressure of pure material should be less than about 67 Pascal (0.5mmHg), preferably less than about 13 Pascal (0.1mmHg) each at 20°C. It may not be strait forward to predict whether a particular compound will work if it is not used in its neat form but as a compound in a composition. If doubts about the volatility in such a composition are expressed it is suggested to evaluate whether the compound in the composition as intended for use does vaporize sufficiently to achieve the desired effect. This evaluation can be made by a 5 (or better 10) person panel or by vapour head evaluation e.g. with gas chromatography.

Lotion and application of the lotion

A "lotion" is a composition added to the tissue preferably at the converting phase in order to improve its softness and/or smoothness and has a smoothening effect when some of the lotion is transferred from the tissue to the user's skin upon use of the paper tissue article. The lotion may comprise tissue softening and/or debonding agents, emollients, immobilizing agents and mixtures thereof. Suitable softening and/or debonding agents include quaternary ammonium compounds, polysiloxanes, and mixtures thereof. Suitable emollients include propylene glycol, glycerine, triethylene glycol, spermaceti or other waxes, petrolatum fatty acids, fatty alcohols and fatty alcohol ethers having from 12 to 28 carbon atoms in their fatty acid chain, mineral oil, namely silicone oil e.g. dimethicone and isopropyl palmitat, and mixtures thereof. Suitable immobilizing agents include waxes, fatty alcohols, fatty acids, e.g. ceresin wax, microcrystalline wax, petroleum waxes, fisher tropsh waxes, paraffin waxes, stearyl alcohol and paraffins, polyhydroxy fatty acid esters, polyhydroxy fatty acid amides, and mixtures thereof. In most cases the lotions contain at least one immobilizing agent and an emollient. Lotions can be emulsions or dispersions. Other optional components include perfumes, antibacterial actives, antiviral actives, disinfectants, pharmaceutical actives, film formers, deodorants, opacifiers, astringents, solvents and the like. Particular examples of lotion components according to the present invention include thymol, chamomile extracts, aloe vera, calendula officinalis.

According to the present invention the volatile cooling sensate can be incorporated into the lotion. This has the added advantage that the cooling sensate is transferred to the skin in the region where the cooling is mostly intended and when the tissue product is used as a handkerchief the volatility of the sensate allows highly effective transport of the sensate to receptor sites of the mucous skin in the nasal cavity. According to the present invention the cooling sensate can be included in the lotion irrespective of the lotion deposition on the fibrous tissue. However the more effective the lotion deposition for lotion transfer the more effective will the cooling sensate perform as well.

In this context it is particularly preferred that the lotion comprising a cooling sensate has no olfactory disadvantage to the same lotion without the sensate. In other words there should not be a substantially different smell of the lotion due to the sensate component. This is also referred to as subliminal usage of a cooling sensate and would allow quantifying how much of an otherwise objectionably smelling cooling sensate could be used without causing such objection. This can be evaluated by triangular sensory smell analysis according to DIN 4120, of January 1995 when comparing tissue samples carrying the lotion with and without the cooling sensate. Of course such comparison requires proper setting according to DIN 10962 (requirements for sensor test rooms), education of the test individuals and recognition of standard compositions according to DIN 10961 and DIN 10964 as well as education of test individuals and ranking of intensity differences (including menthol, camphor evaluations). Comparisons resulting in insignificant differences (or even identity) will be acceptable in the context of the present invention, however also comparisons where a deviation of less than 20% of the ranking scale can be found are considered acceptable and would usually be referred to as slightly recognizable smell. Compositions comprising cooling sensates causing no more than such slightly recognizable smell detection are considered not detectable by smell analysis for the purpose of the present invention.

"Lotion deposit" (or "deposit") is an area of relatively high lotion basis weight. A deposit is defined as an area of tissue comprising lotion with a local lotion basis weight of at least 10g/sqm. Areas on the tissue with lower local basis weights are not part of a deposit. The local basis weight on the tissue is measured as described thereafter.

"Lotion basis weight of the deposits" is the basis weight of lotion, expressed in grams per square meter, within the region of the deposits of lotion on the tissue. This takes only into account the area of the deposits and the amount of lotion within the deposits and is an average value of the deposits measured. The lotion basis weight of the deposits is measured by the method described thereafter.

"Lotion basis weight of the tissue" is the overall basis weight of lotion, expressed in grams per square meter, of lotion on the tissue. The basis weight can be measured by standard methods, e.g. solvent extraction, or calculated from the process conditions (total amount of lotion deposited on the tissue divided by the total area of the tissue).

"Size of deposits" is the average size of the deposits of lotion on the tissue, as measured by the method described thereafter.

"Area of tissue affected by lotion" is an area with a local lotion basis weight of more than 3 g/sqm as determined by the method described herein.

It is desirable to provide a tissue with a lotion able to be transferred easily onto skin during use. The selection of the distribution of the lotion on the tissue, as a multitude of discrete deposits, can enhance the transferability of the lotion from the tissue onto the skin of the user. The higher the basis weight of the lotion within the deposits the higher the availability for transfer to skin upon use. Indeed, with a relatively high local concentration of lotion in discrete deposits, a relatively low amount of lotion remains stuck on the tissue. It is hence desirable to provide discrete deposits with a high local concentration of the lotion in the deposits and a relatively low basis weight of lotion of the tissue. In preferred embodiments of the invention, the lotion basis weight of the deposits is at least 11 g/sqm, 13 g/sqm, 15g/sqm, 17g/sqm, 20g/sqm, 25g/sqm or most preferably more than 30g/sqm, while the basis weight of lotion of the tissue is equal or less than 9 g/sqm, less than 6g/sqm, less than 4.5g/sqm, 3.0g/sqm or most preferably less than 2g/sqm.

The application of the lotion can be done via the use of rotating applicator surfaces from which the lotion is expulsed to impact a fibrous tissue. The temperature of the rotating surface needs to match the characteristics of the lotion, in order to ensure balancing two phenomena: First, the expulsion disperses the lotion into a stream or cloud of droplets. The size and density of the stream of droplets can be controlled by temperature at which, the lotion leaves the rotating applicator surface. Second, the droplets are expulsed in a liquid or quasi-liquid form and are in a solid or quasi-solid form when they impacted the tissue to prevent their penetration into and absorption within the tissue. The droplets have a tendency to stay immobilized at the surface of the tissue supporting the desired high transferability of the lotion. Examples of rotating applicator units capable to deliver discrete deposits of lotion are described in WO-A-02/34519 or WO-A-02/234520. A commercially available rotary spray application system RFT-Compact-III with applicator heads for the tissue and textile industry are available from Weitmann & Konrad GmbH & Co KG, Leinfelden-Echterdingen, Germany.

Example of lotion composition including cooling sensate on a handkerchief tissue paper product:

The following composition (given in weight percent) has been found of particular efficacy on disposable handkerchiefs. It is hypothesized that the lotion is particularly suited to provide the desired lotion deposits and distribution. Also the particular cooling sensate provides significant perception of decongestion when used by individuals having a cold or other rhinological discomfort.

| | |
|---|---|
| Stearyl Alcohol CO1897 * | 39.9% |
| Petrolatum Snowwhite V28EP ** | 29.9% |
| Mineral oil Carnation ** | 29.9% |
| 2-Isopropyl-N,2,3-trimethylbutyramide | 0.3% |
| (WS23)*** | |
| * Available from Procter&Gamble Chemicals, Cincinnati, USA<br>** Available from Crompton Corporation<br>*** Available from Millennium Specialty Chemicals | |

The above formulation was applied equally to both outer surfaces of a tissue paper product. Total add-on level was 6g/sqm, 3g on each outer surface, using a rotary surface application method to create discrete lotion deposits according to the preferred embodiment of the invention.

The paper tissue used was a conventional wet pressed, homogeneous, dry creped tissue essentially of wood pulp fibers with a basis weight of about 15.4 g/sqm. The wood pulp fibers had a composition of about 40% Northern Softwood Kraft fibers and 60% Eucalyptus fibers. Following the papermaking, four sheets of paper are combined in an off line combining operation and rewound into a parent roll. The pre-combined 4-ply parent roll is subsequently converted into a 4-ply tissue product. The 4-ply parent roll is unwound and subjected to calendaring between two smooth steel calender rolls followed by high pressure embossing to achieve ply bonding. The majority of the tissue paper remains unaffected by the high pressure embossing. Finally the tissue was cut in machine direction, followed by cutting in cross direction into sheets of approximately 21cm x 21cm, folded, stacked into stacks of 9 handkerchiefs and packed into individual handkerchief pocket packs. The 4-ply paper tissue product obtained by the above described process had a basis weight of approximately 60g/sqm (not including lotion), a thickness of 0.27mm, a machine direction strength of 1280 g/2.54cm, a cross direction strength of 610 g/2.54cm, and a wet burst of about 200g. It contained a wet strength agent and a dry strength agent.

The product was then submitted to a consumer use test together with a comparative tissue product treated with the same lotion but without the cooling sensate (increased percentages by 0.1% each) with the same add-on level on the tissue. A significantly higher proportion of panelists claimed to perceive a refreshed feeling and to be able to breathe more freely when using the handkerchiefs of this example compared to the comparative product. It is believed that the cooling effect of WS-23 is causing the decongestive sensation benefits.

Test methods:

Lotion basis weight of the tissue, as an average lotion basis weight can either be determined by calculating the basis weight from material consumption of lotion and surface are of tissue. Alternatively a solvent extraction method can be used to measure the lotion basis weight of the tissue in the absence of knowing material consumption values or to reconfirm that no substantial amounts of lotion are lost during processing. In this method a representative sample of about 2g of the lotion treated tissue is used. First the surface area of the sample is determined. Then the lotion is extracted by Accelerated Solvent Extraction (ASE) using a model ASE 200, available from Dionex Corp., USA. The conditions should be such that all lotion ingredients are extracted. The solvent is evaporated and the residue is determined gravimetrically. The lotion basis weight of the tissue is then calculated as weight of the extract in grams divided by the surface area of the sample.

Care should be taken when selecting the solvent for this method to be substantially able to dissolve all components of the lotion. In cases where the lotion is insufficiently soluble in this solvent to perform a quantitative extraction, an alternative solvent has to be chosen that is suitable to quantitatively extract the lotion.

<u>Method for quantifying the lotion basis weight of the deposits:</u>

This method allows determination of local lotion basis weight (LLBW), lotion basis weight of the sample (LBWS), lotion basis weight of the deposits (LBWD), Area affected by deposits (AAD), Area affected by lotion (AAL), and average deposit size (ADS).

The local lotion basis weight is determined by scanning IR/NIR (infrared or near infrared) spectroscopy in transmission mode (absorption spectroscopy) using a Perkin Elmer Spectrum Spotlight 300 instrument in combination with Spotlight software version 1.1.0 B38.

The following procedure is applicable to lotions containing linear hydrocarbon components of repeated - (CH2) - units. Adaptation of the procedure may become necessary if the lotion is composed mostly or entirely of other materials. Such adaptations will depend on the lotion composition and will usually be apparent to those skilled in the art.

The measurements are done with samples representative for the tissue. A 5 x 5 mm sample (or larger) is placed on the sample holder, which is mounted on a XY table and the spectral area used for analysis is scanned at a spatial resolution of 25 $\mu$m in both x and y dimension. For the analysis of materials containing linear chains of -CH2- groups the region between 4000cm-1 and 4500cm-1 is scanned and the range between 4296cm-1 (W1) and 4368cm-1 (W2) is used for analysis. At least 16 scans are taken at a resolution of 1 cm-1. If more than 16 scans are used, care needs to be taken that the sample does not change structure as a result of heating up.

Next, a map of the local basis weight of the sample is generated. The integrated absorption between W2 and W1 and above a sloping linear baseline is determined for each pixel of 25$\mu$m x 25$\mu$m using the ChemiMap menu of the software. The baseline is defined by the absorbency at W1 and W2. The two base points option is chosen in the ChemiMap menu of the software and set at W1 and W2. Start and end point of the integration are also set at W1 and W2. The scaling factor is set to a value V1 which is defined as: V1 = F* DW where F is the factor described below and DW=W2-W1 is the delta in wave numbers between the upper (W2) and the lower (W1) wave number in cm$^{-1}$.

The scaling with the factor DW transforms the average absorbance above the baseline within the wave number range W1 to W2 into an integrated absorption above the baseline. The factor F translates the integrated absorption into local basis weight in g/sqm.

The file, which is generated with the ChemiMap command, contains the local basis weight for each pixel of 25$\mu$m x 25$\mu$m in area. The file is saved as a text file (.txt format) and also as a bitmap (.bmp format) in 8 bit grey scale format. The text file is imported into EXCEL and the first row and first column are removed (they do not contain image data, but position data). The resulting data are representing the array of pixels of local basis weight in g/sqm. The maximum (MaxLBW) and minimum (MinLBW) value, as well as the average (AvgLBW) of the whole dataset is calculated in EXCEL.

The bitmap file (.bmp file) is imported into AnalySIS image analysis software for further processing (Analysis Pro version 3.1 (build 508), available from Soft Imaging GmbH, Germany). The imported grey scale file is still in RGB format with all three color channels set equal (in 8 bit resolution). In AnalySIS the file is color separated to extract one of the three identical color channels (red). The resulting file is now scaled from G=0 to G=255, G=0 representing the minimum value (MinLBW) of the original spotlight data and 255 representing the maximum value (MaxLBW) of the original spotlight data. The image is calibrated in x-y by setting the pixel size in x and y dimension to match the original sample. The image is rescaled in z-direction to display the local basis weight values in g/sqm but all calculations within AnalySIS have to be made in the G=0 to G=255 scale. The G values can be easily transformed into local lotion basis weight numbers by the following relationship:

$$LLBW=A*(G+OFFSET), \quad \text{where} \quad A= \quad (MaxLBW-MinLBW)/255 \quad \text{and}$$

$$OFFSET=(255*MinLBW)/(MaxLBW-MinLBW)$$

The G values can be easily transformed into local lotion basis weight numbers (LLBW) by the following relationship: G=(LLBW/A) - OFFSET

Calculation of the lotion basis weight of the deposits: The average value of all local lotion basis weight datapoints above 10g/sqm can be calculated from the EXCEL datafile.

The area of tissue affected by lotion is calculated in Analysis by setting a lower threshold at the G value equivalent to 3g/sqm and calculating the area above that threshold. The setting "holes not filled" is used. The area of the deposits is similarly determined by setting the threshold at a G value equivalent to 10g/sqm (10g/sqm equals G=10/A - OFFSET).

If deposits are defined to have a certain minimum and / maximum area is set as a filter. The area percentage of deposits larger than a certain area is calculated by dividing the area of the deposits calculated without area filter, divided by the area of the deposits calculated with area filter.

The factor F to convert integrated absorption values into local lotion basis weight values is determined by the following procedure: A representative set of calibration samples of known average lotion basis weight is scanned in the spectral range used for the analysis as described above and analyzed for integrated peak area between W1 and W2 (4296cm-1 and 4368cm-1 for mostly hydrocarbon like materials). The integrated peak area is obtained from the procedure above if the factor F is set equal to 1. The dataset is then imported to EXCEL and the average pixel value of this dataset is calculated. As the factor F was set equal to 1 this value is equal to the mean integrated peak area (AIPA) of the sample in the wave number range W1 to W2. The factor F is then calculated as F=1/slope of a linear least square fit through the origin of the plot of AIPA vs. average lotion basis weight of the sample. Calibration samples to determine the factor F can either be prepared or an existing lotioned sample can be used. If an existing sample is used the lotion basis weight can be determined by extraction. An example for such a procedure is given below. Examples for how the factor F is determined by analyzing an existing sample (market product) and by preparing calibration samples is also given below. It is important, that the absorbency in the wavelength range used for analysis should never exceed about 1 to ensure a linear correlation between the infrared signal and the local lotion basis weight

Determination of factor F by preparing calibration samples

Preparation of calibration samples: A suitable piece of the substrate of known area, weight and basis weight is evenly treated with lotion, preferably by evenly spraying the molten lotion onto the tissue. A suitable type of equipment is a hot wax cartridge spray gun type MK-DUO Line Art.No. 140101, available from MK Heißwachstechnik GmbH, Aichach, Germany. After the application, the lotion is equilibrated in the sheet by placing the sample in an oven at a temperature of about 10°C above the mp (or at a temperature suitable to allow for sufficient equilibration of the lotion in the sheet). For relatively low viscosity samples equilibration for about an hour is sufficient. The sample is then cooled down to room temperature and equilibrated for moisture content at 23°C (+-1°C) and 50% (+-2%) relative humidity and weighed again. The lotion basis weight of that sample [in g/sqm] is then calculated as (sample weight after lotion treatment [in grams] - sample weight before lotion treatment [in grams]) divided by area of the sample [in sqm]. The samples are then analyzed by the procedure described above to determine the factor F. Preferably, calibration samples are prepared in a range of lotion basis weights that include the range to be measured.

Determination of Factor F for a market product: The basis weight of the sample is determined by a standard procedure. The sample is then analyzed by the procedure described above for the average integrated peak area between 4296cm-1 and 4368cm-1. The sample is then extracted by the procedure described below to determine the lotion add-on. The Factor F is then calculated as

$$\text{Factor F} = \text{lotion basis weight [g/sqm]} / \text{average integrated peak area}$$

If the lotion does not contain a sufficient amount of linear hydrocarbon like material, or the substrate contains materials that do not allow for a quantification of lotion between 4296cm-1 and 4368cm-1, a different wave number range in the infrared or near infrared range has to be identified that is suitable to quantify the lotion by IR spectroscopy. Any wave number range with a linear correlation between integrated absorption coefficient above base line and lotion basis weight can be used. If more than one possible wave number range can be identified, the range with the best signal to noise ratio is used. Whenever the lotion is based on linear hydrocarbon like materials with CH2 groups the absorption band between 4296cm-1 and 4368cm-1 should be used.

**Claims**

1.  A facial tissue, bathroom tissue, toilet tissue, or disposable handkerchiefs comprising a fibrous tissue **characterized in that** said tissue comprises:

    - fibers suitable for the final intended use, preferably including cellulose fibers; and,
    - a volatile cooling sensate comprising 2-Isopropyl-N,2,3- trimethylbutyramide as volatile cooling sensate, said volatile cooling sensate excluding menthol and camphor and being reactive on human mucous skin by causing a cold feeling sensation without directly causing a change in skin temperature.

2.  The facial tissue, bathroom tissue, toilet tissue, or disposable handkerchiefs according to claim 1 further comprising a transferable lotion.

3.  The facial tissue, bathroom tissue, toilet tissue, or disposable handkerchiefs according to claim 1 or 2 **characterized in that** said volatile cooling sensate is incorporated in a transferable lotion composition, said transferable lotion composition being capable of transferring from the tissue to skin when the tissue comes into contact with skin.

4.  The facial tissue, bathroom tissue, toilet tissue, or disposable handkerchiefs according to claim 2 or 3 wherein said lotion is present in discrete deposits on at least one surface of said tissue and said lotion basis weight in said deposits is at least 11 g/sqm, preferably at least 15 g/sqm, more preferably at least 25g/sqm, and the basis weight of lotion on the tissue is equal or less than 9 g/sqm, preferably less than 6g/sqm, and more preferably less than 3.0g/sqm.

5.  The facial tissue, bathroom tissue, toilet tissue, or disposable handkerchiefs according to claim 4 wherein there are at least 2 discrete deposits per square cm of tissue.

6.  The facial tissue, bathroom tissue or disposable handkerchief comprising a fibrous tissue according to any of the proceeding claims wherein said tissue comprises at least 50%, preferably 80%, and more preferably 90% of cellulose fibers by weight of said fibers of said tissue.

7.  The facial tissue, bathroom tissue, toilet tissue or disposable handkerchief according to any of the preceding claims wherein the amount of said volatile cooling sensate is such that it is not detectable by smell analysis as defined herein.

**Patentansprüche**

1.  Kosmetiktuch, Waschtuch, Toilettenpapier oder Einwegtaschentücher, umfassend ein Fasergewebe, **dadurch gekennzeichnet, dass** das Gewebe umfasst:

    - Fasern, die für den endgültigen bestimmungsmäßigen Gebrauch geeignet sind, vorzugsweise Cellulosefasern beinhaltend; und
    - eine flüchtige sinnlich wahrgenommene Kühlsubstanz, umfassend 2-Isopropyl-N,2,3-trimethylbutyramid als flüchtige sinnlich wahrgenommene Kühlsubstanz, wobei die flüchtige sinnlich wahrgenommene Kühlsubstanz Menthol und Kampfer ausschließt und auf menschlicher Schleimhaut reaktiv ist, indem sie eine Kältegefühlempfindung bewirkt, ohne direkt eine Änderung der Hauttemperatur zu bewirken.

2.  Kosmetiktuch, Waschtuch, Toilettenpapier oder Einwegtaschentücher nach Anspruch 1, ferner eine übertragbare Lotion umfassend.

3.  Kosmetiktuch, Waschtuch, Toilettenpapier oder Einwegtaschentücher nach Anspruch 1 oder 2, **dadurch gekennzeichnet, dass** die flüchtige sinnlich wahrgenommene Kühlsubstanz in eine übertragbare Lotionszusammensetzung eingeschlossen ist, wobei die übertragbare Lotionszusammensetzung in der Lage ist, von dem Gewebe auf Haut überzugehen, wenn das Gewebe mit Haut in Kontakt kommt.

4.  Kosmetiktuch, Waschtuch, Toilettenpapier oder Einwegtaschentücher nach Anspruch 2 oder 3, wobei die Lotion in separaten Ablagerungen auf mindestens einer Oberfläche des Gewebes vorhanden ist und das Basisgewicht der Lotion in den Ablagerungen mindestens 11 g/qm, vorzugsweise mindestens 15 g/qm, mehr bevorzugt mindestens 25 g/qm beträgt und das Basisgewicht der Lotion auf dem Gewebe gleich oder weniger als 9 g/qm, vorzugsweise weniger als 6 g/qm und mehr bevorzugt weniger als 3,0 g/qm beträgt.

5. Kosmetiktuch, Waschtuch, Toilettenpapier oder Einwegtaschentücher nach Anspruch 4, wobei mindestens 2 separate Ablagerungen pro Quadratzentimeter Gewebe vorhanden sind.

6. Kosmetiktuch, Waschtuch oder Einwegtaschentuch, umfassend ein Fasergewebe nach einem der vorstehenden Ansprüche, wobei das Gewebe zu mindestens 50 %, vorzugsweise 80 % und mehr bevorzugt 90 % Cellulosefasern, bezogen auf das Gewicht der Fasern des Gewebes, umfasst.

7. Kosmetiktuch, Waschtuch, Toilettenpapier oder Einwegtaschentuch nach einem der vorstehenden Ansprüche, wobei die Menge der flüchtigen sinnlich wahrgenommenen Kühlsubstanz derart ist, dass sie nicht durch Geruchsanalyse, wie hierin definiert, wahrnehmbar ist.


**Revendications**

1. Lingette pour le visage, papier hygiénique, papier toilette, ou mouchoirs jetables comprenant un papier absorbant fibreux **caractérisé en ce que** ledit papier absorbant comprend :

   - des fibres appropriées pour l'utilisation finale prévue, incluant de préférence des fibres de cellulose ; et,
   - un agent sensoriel de refroidissement volatil comprenant du 2-isopropyl-N,2,3-triméthylbutyramide en tant qu'agent sensoriel de refroidissement volatil, ledit agent sensoriel de refroidissement volatil excluant le menthol et le camphre et étant réactif sur la peau de muqueuse humaine en causant une sensation de froid sans causer directement un changement de température de la peau.

2. Lingette pour le visage, papier hygiénique, papier toilette, ou mouchoirs jetables selon la revendication 1, comprenant en outre une lotion transférable.

3. Lingette pour le visage, papier hygiénique, papier toilette, ou mouchoirs jetables selon la revendication 1 ou 2, **caractérisés en ce que** ledit agent sensoriel de refroidissement volatil est incorporé dans une composition de lotion transférable, ladite composition de lotion transférable étant susceptible de se transférer du papier absorbant à la peau lorsque le papier absorbant vient en contact avec la peau.

4. Lingette pour le visage, papier hygiénique, papier toilette, ou mouchoirs jetables selon la revendication 2 ou 3, dans lesquels ladite lotion est présente en dépôts distincts sur au moins une surface dudit papier absorbant et la masse surfacique de ladite lotion dans lesdits dépôts est d'au moins 11 g/m$^2$, de préférence au moins 15 g/m$^2$, plus préférablement au moins 25 g/m$^2$, et la masse surfacique de lotion sur le papier absorbant est égale ou inférieure à 9 g/m$^2$, de préférence inférieure à 6 g/m$^2$, et plus préférablement inférieure à 3,0 g/m$^2$.

5. Lingette pour le visage, papier hygiénique, papier toilette, ou mouchoirs jetables selon la revendication 4, dans lesquels il y a au moins 2 dépôts distincts par cm carré de papier absorbant.

6. Lingette pour le visage, papier hygiénique, papier toilette, ou mouchoir jetable comprenant un papier absorbant fibreux selon l'une quelconque des revendications précédentes, dans lequel ledit papier absorbant comprend au moins 50 %, de préférence 80 %, et plus préférablement 90 % de fibres de cellulose en poids desdites fibres dudit papier absorbant.

7. Lingette pour le visage, papier hygiénique, papier toilette, ou mouchoir jetable selon l'une quelconque des revendications précédentes, dans lequel la quantité dudit agent sensoriel de refroidissement volatil est telle qu'elle n'est pas détectable par une analyse d'odeur telle que définie ici.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5059282 A, Ampulski
- EP 1250940 A
- EP 1250941 A
- US 5451404 A
- US 5266592 A
- DE 2608226 A
- DE 2458562 A
- WO 0234519 A
- WO 02234520 A

### Non-patent literature cited in the description

- **HERBERT HENSEL.** Thermal sensation and thermo-receptors in man. Charles C. Thomas, 1982
- **H.R. WATSON et al.** *Journal of the Society of Cosmetic Chemist,* 1978, vol. 29, 185-200